# EUROPEAN PATENT APPLICATION

(11) **EP 4 810 123 A2**
(43) Date of publication of application: **23.09.2026**
(21) Application number: 26196378.9
(22) Date of filing: 01.11.2022
(51) Int. Cl.: A61P 43/00

(54) **MEDIUM CHAIN DICARBOXYLIC ACIDS FOR THE TREATMENT AND PREVENTION OF DISEASES AND CONDITIONS**

(30) Priority: 02.11.2021 US 202163274674 P
(62) Document number(s) of the earlier application(s) in accordance with Art. 76 EPC: 22822722.9 / 4 426 281
(71) Applicant: University of Pittsburgh- Of the Commonwealth System of Higher Education, Pittsburgh PA 15260 (US)
(72) Inventor: SIMS-LUCAS, Sunder, Pittsburgh, 15260 (US); GOETZMAN, Eric S., Pittsburgh, 15260 (US)
(74) Representative: HGF

(57) **Abstract**

A method including administering a therapeutically effective amount of a therapeutic agent to a subject for improving exercise performance, treating obesity, fatty liver, rhabdomyolysis, Leigh syndrome, mitochondrial Complex I deficiency, fatty acid oxidation disorders, lactic acidosis, metformin toxicity, ischemic acute kidney injury, or nephrotoxin-induced kidney injury, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.

## Description

This application claims the benefit of U.S. Provisional Appl. No. 63/274,674, filed November 2, 2021, which is incorporated herein by reference.

### BACKGROUND

Metabolism, in its simplest definition, is the process by which the foods you consume are converted into cellular energy. Metabolism is an underlying component of most human diseases. For example, cancer rewires metabolism in order to fuel rapid expansion and growth of tumor cells. Further, chronic diseases of the brain, heart, liver, and kidney all have strong metabolic components that contribute to disease pathogenesis. Even many acute conditions such as sepsis, acute kidney injury, traumatic brain injury, and burn injuries are characterized by metabolic changes. Finally, in developed countries such as the United States, diseases related to chronic overnutrition are an ever-growing healthcare burden. Despite decades of intensive research on obesity and metabolism, there is still no effective therapy to help the millions of obese people in our country lose weight in a healthy manner. One attractive strategy for weight loss is to increase basal metabolic rate. To date, however, this strategy has failed. After normalizing for body mass, humans appear to be hardwired to stay within a narrow band of metabolic rate throughout adulthood. Drugs that can effectively move this needle have been elusive.

Acute kidney injury (AKI) most commonly occurs in the hospital setting. Hospital-acquired AKI accounts for 22% of all cases worldwide, and nearly 50% of critically ill inpatients are estimated to suffer from AKI. AKI is associated with high rates of morbidity and mortality, and causes 2 million deaths per year. The hospitalization cost of AKI treatment in the US per year is approximately $24 billion. While the kidney may recover, the patients are at a higher risk for subsequently developing chronic kidney disease (CKD); other times, the acute injury is so severe that there is no kidney recovery and ultimately end stage renal disease (ESRD). Patients that progress to CKD have hospitalization costs of approximately 100 billion dollars a year in the US.

Currently, health care providers lack a definitive and effective treatment for AKI or
even sufficient interventions to decrease the risk of progression to CKD after AKI. The best strategies currently focus on prevention, early diagnosis and early interventions aimed at managing the underlying etiologies and complications of AKI. Damage to the proximal tubules, a hallmark of AKI, alters metabolic function of the proximal tubules and the kidney, contributing to fibrosis and inflammatory injury to the kidney. Although proximal tubule de-differentiation to repair the damaged tubule is critical to recovery after AKI, the mechanisms that govern this process and reverse it are still elusive.

In addition to the more common conditions of cancer, obesity, and AKI, there are also a myriad of rare disorders with dysfunctional metabolism that currently lack therapeutic options. Examples include mitochondrial disorders such as Leigh syndrome and long-chain fatty acid oxidation disorders. Patients with genetic defects in the mitochondrial enzymes that chain-shorten long-chain fatty acids exhibit cardiac defects, intolerance to fasting, and muscle attacks called rhabdomyolysis that will plague them throughout their lives. Finally, besides genetic disorders, there are acquired forms of mitochondrial dysfunction, such as that induced by metformin overdose. Effective therapies are lacking for all of these diseases and disorders.

### SUMMARY

Disclosed herein is a method comprising administering a therapeutically effective amount of a therapeutic agent to a subject for treating obesity, fatty liver, rhabdomyolysis, Leigh syndrome, mitochondrial Complex I deficiency, fatty acid oxidation disorders, lactic acidosis, metformin toxicity, ischemic acute kidney injury, or nephrotoxin-induced kidney injury, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.

Also disclosed herein is a method comprising administering a therapeutically effective amount of a therapeutic agent to a subject in need of a therapeutic agent that bypasses mitochondrial fatty acid oxidation and stimulates peroxisomal fatty acid oxidation, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.

Further disclosed herein is a method comprising administering a therapeutically effective amount of a therapeutic agent to a subject for improving exercise performance, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.

The foregoing will become more apparent from the following detailed description, which proceeds with reference to the accompanying figures.

### BRIEF DESCRIPTION OF THE DRAWINGS

FIGS. 1A-1C. Feeding mice DCAs causes a dramatic shift in the post-translational modification (PTM) landscape. FIG. 1A) DC12 was fed to wild-type mice at 10% w/w for 7 days, resulting in a massive increase in lysine succinylation (Suc-Lys) PTMs in both liver and kidney, as visualized by western blotting. ND=normal diet. FIG. 1B) Mice were fed 10% DC12 for 0 to 7 days; some mice underwent a 7-day "washout" after 7 days on DC12. FIG. 1C) Feeding the odd-chain DC11 for 7 days at 10% w/w causes massive increase in lysine glutarylation (Glu-Lys).
FIGS 2A-2C. DC12 induces equal lysine succinylation in WT versus Sirt5·/·mouse liver peroxisomes. FIG. 2A) Livers from N=3 mice fed DC12 for 7 days, separated by Histodenz gradient centrifugation to yield pure peroxisomes and blotted with anti-succinyllysine (SucLys). ND lanes = one control mouse of each genotype fed normal diet. Peroxisome fraction is positive for peroxisome marker PMP70 but negative for mitochondrial Tim23 except for WT sample #2 which had minor mitochondrial contamination. FIG. 2B) Densitometry from panel (FIG. 2A) showing total SucLys signal normalized to PMP70. FIG. 2C) Liver homogenates from normal diet (ND) and Sirt5KO mice (N=4) were subjected to quantitative site-level mass spectrometry for succinylated peptides. The Y-axis is Log2 fold-change for each peptide relative to wild-type (WT) on ND. Each dot is an individual peptide. SirtSKO on ND show very little change from WT, and DC12 feeding induces very high fold-changes in succinylation in both genotypes.
FIG. 3. DC12 treatment mitigates ischemic kidney injury. Normal diet or
   DC12-fed male mice were subjected to ischemic AKI. A,B) Morphologically, DC12 reduced kidney damage as observed by H&E staining. Tubular dilation (arrows), debris, or proteinaceous casts are indicated (*). C, D) DC₁₂ markedly reduced expression of the injury marker NGAL (red) in proximal tubules (indicated by tubule marker LTL, green). E,F) DC12-fed mice had lower serum creatinine and BUN, which are markers of kidney dysfunction. **p<0.05.*
FIGS. 4A-4C. Effects of injury and DC12 on protein succinylation PTMs. N=4 mice were subjected to AKI± 7 days of 10% DC12. Contralateral (non-injured kidneys) served as control. Quantitative mass spectrometry was used to profile lysine succinylation PTMs at the protein and site-level. FIG. 4A) In control-fed mice, injury dramatically reduced succinylation (blue are significantly reduced sites, red are significantly increased sites). FIG. 4B) In non-inured kidneys, DC12 massively increased succinylation (again, red at up, blue are down-regulated). FIG. 4C) Succinylation of individual lysine-containing peptides from four key peroxisomal proteins, normalized to control diet non-injured levels.
FIGS. 5A-5B. DC12 treatment mitigates cisplatin kidney injury. Normal diet or DC12-fed (7 days pre-treatment + 3 days post-treatment) male 129 WT mice were injected i.p. with 18mg/kg cisplatin and sacrificed at day 3. (FIG. 5A) Reduction of serum bicarbonate by cisplatin-AKI was mitigated in DC12 treated mice. (FIG. 5B) Renal tubular injury by cisplatin is mitigated in DC12 treated mice. Magnified images for dotted boxed areas are shown. Tubular dilation, debris, or proteinaceous casts are indicated(*) n=6-7, mean±SEM, t-test, *p<0.05, Scale bars: 50um
FIG. 6. DC8 causes robust kidney specific succinylation. DC8, DC10 and DC12 were fed to wild-type mice at 10% w/w for 7 days. DC8 resulted in a dramatic increase in lysine succinylation (Suc-Lys) PTMs in the kidney but not the liver, as visualized by western blotting.
   DC10 showed a large increase in Suc-Lys PTMs in kidney and a moderate upregulation in the liver. DC12 resulted in a moderate increase in kidney Suc-Lys PTMs and a large increase in the liver.
FIGS. 7A-7F. DC8 treatment shows enhanced protection against ischemic kidney injury. Normal diet or DC8-fed male mice were subjected to ischemic AKI. FIGS. 7A,7B) Morphologically, DC8 reduced kidney damage as observed by H&E staining. Tubular dilation (arrows), debris, or proteinaceous casts are indicated (*). FIGS. 7C, 7D) DC8 markedly reduced expression of the injury marker NGAL (red) in proximal tubules (indicated by tubule marker LTL, green). FIGS. 7E,7F) DC8-fed mice had lower serum creatinine and BUN, which are markers of kidney dysfunction.
   *****p*<0.0001.
FIGS. 8A-8G. DC12 reduces fat mass and increases whole-body respiration. FIG 8A, 8B) Mice were fed DC12 for 5 weeks. Body weight and food weights were measured 3 times per week. DC12 reduced body weight without changing food intake. FIG8C) DC12 does not reduce muscle strength. Forelimb peak force was measured with a grip strength meter and normalized to body weight. FIG 8D, 8E) DC12 specifically reduces fat mass while not altering lean mass, as determined in mice fed DC12 for 5 weeks or 9 weeks and assessed by EchoMRI. At sacrifice, the mass of the intra-abdominal fat pad (epididymal) was significantly reduced by DC12 (FIG 8F) and liver fat (triacylglycerol, or TAG) was also reduced (FIG 8G).
FIGS. 9A-9D. DC12 increases whole-body energy expenditure and respiratory exchange ratio. Indirect calorimetry indicated increased whole-body respiration as measured in the CLAMS apparatus over 72 hrs, for both oxygen consumed (FIG 9A) and carbon dioxide produced (FIG 9B). The ratio of these two parameters (respiratory exchange ratio, RER) indicates a shift toward a higher RER during the dark period, consistent with enhanced carbohydrate oxidation (FIG 9C). The calculated energy expenditure in the DC12 group was higher (FIG 9D).
FIGS. 10A-10D. DC12 protects against lactic acidosis. Wildtype mice were maintained on DC12 diet for 7 days, then run on a treadmill to exhaustion. Blood lactate was significantly reduced at exhaustion (FIG 10A). Then, mice fed DC12 were tested for protection against metformin overdose, which is clinically known to induce a lethal lactic acidosis. After a single 400 mg/kg dose of metformin, 9/11 mice on control diet died, while 0/12 mice on DC12 diet died (FIG 10B). At 2 hr post-metformin injection, just prior to the onset of death in the control group, blood glucose levels were twice as high as control in the DC12-fed mice while lactate levels were 50% lower (FIG 10C, D).
FIGS. 11A-11E. DC12 and DC10 improve functioning in long-chain acyl-CoA dehydrogenase (LCAD) knockout mice, a mouse model of mitochondrial long-chain fatty acid oxidation disorders. After two weeks on 10% DC12 diet, LCAD knockout mice showed improved fasting glucose (FIG 11A), improved forelimb grip strength (FIG 11B), and improved spontaneous locomotion with less time spent resting (FIG 11C, 11D). Finally, a single dose of DC10 significantly lowered blood lactate after an acute exercise stress test on a treadmill (FIG 11E).
FIG. 12A-D. While dicarboxylic fatty acids 8-12 carbons in length occur naturally in small amounts in the mammalian liver, triglycerides of these fatty acids do not occur naturally, and represent novel therapeutic formulations of dicarboxlic acids. FIG 12A and 12B illustrate structures of the triglyceride forms of DC12 and DC8, respectively. Similar triglycerides could be synthesized for other dicarboxylic fatty acids. Also, addition of sodium ions (FIG 12C) allows for greater solubility. As proof of concept, the triglyceride form of DC8 has been synthesized at the University of Pittsburgh to high purity as shown by high-performance liquid chromatography (FIG 12D).
FIG. 13. Similarly, novel therapeutic formulations of dicarboxylic fatty acids can be created by chemical synthesis of metabolic intermediates produced during the degradation of straight-chain dicarboxylic acids. Shown in FIG 13 are the intermediates in DC8 degradation. Similar intermediates can be synthesized for other chain lengths of straight-chain dicarboxylic fatty acids. Also, these intermediates can be conjugated to glycerol to make triglycerides as shown in FIG 12.
FIG. 14. DC8 treatment was also protective against a chronic kidney disease model of interstitial fibrosis. As was evidenced in the control fed diet 14A and A' when the kidney was subjected to unilateral ureteral obstruction (UUO) that there was a significant amount of trichrome positive fibrosis in the interstitium (blue). This contrasted with the DC8 fed animals 14B and B' where the amount of interstitial fibrosis was dramatically decreased.

### DETAILED DESCRIPTION

### Terminology

The following explanations of terms and methods are provided to better describe the present compounds, compositions and methods, and to guide those of ordinary skill in the art in the practice of the present disclosure. It is also to be understood that the terminology used in the disclosure is for the purpose of describing particular embodiments and examples only and is not intended to be limiting.

"Administration" as used herein is inclusive of administration by another person to the subject or self-administration by the subject.

An "animal" refers to living multi-cellular vertebrate organisms, a category that includes, for example, mammals and birds. The term mammal includes both human and non-human mammals. Similarly, the term "subject" includes both human and non-human subjects, including birds and non-human mammals. Illustrative non-human mammals include animal models (such as mice), non-human primates, companion animals (such as dogs and cats), livestock (such as pigs, sheep, cows), as well as non-domesticated animals, such as the big cats. The term subject applies regardless of the stage in the organism's life-cycle. Thus, the term subject applies to an organism *in utero* or *in ovo,* depending on the organism (that is, whether the organism is a mammal or a bird, such as a domesticated or wild fowl).

The term "co-administration" or "co-administering" refers to administration of a compound disclosed herein with at least one other therapeutic agent or therapy within the same general time period, and does not require administration at the same exact moment in time (although co-administration is inclusive of administering at the same exact moment in time). Thus, co-administration may be on the same day or on different days, or in the same week or in different weeks. In some embodiments, the co-administration of two or more agents or therapies is concurrent. In other embodiments, a first agent/therapy is administered prior to a second agent/therapy. Those of skill in the art understand that the formulations and/or routes of administration of the various agents or therapies used may vary. The appropriate dosage for co-administration can be readily determined by one skilled in the art. In some embodiments, when agents or therapies are co-administered, the respective agents or therapies are administered at lower dosages than appropriate for their administration alone. Thus, co-administration is especially desirable in embodiments where the co-administration of the agents or therapies lowers the requisite dosage of a potentially harmful (e.g., toxic) agent and/or lowers the frequency of administering the potentially harmful (e.g., toxic) agent. "Co-administration" or "co-administering" encompass administration of two or more active agents to a subject so that both the active agents and/or their metabolites are present in the subject at the same time. Co-administration includes simultaneous administration in separate compositions, administration at different times in separate compositions, or administration in a composition in which two or more active agents are present.

"Inhibiting" refers to inhibiting the full development of a disease or condition. "Inhibiting" also refers to any quantitative or qualitative reduction in biological activity or binding, relative to a control.

The term "subject" includes both human and non-human subjects, including birds and non-human mammals, such as non-human primates, companion animals (such as dogs and cats), livestock (such as pigs, sheep, cows), as well as non-domesticated animals, such as the big cats. The term subject applies regardless of the stage in the organism's life-cycle. Thus, the term subject applies to an organism *in utero* or *in ovo*, depending on the organism (that is, whether the organism is a mammal or a bird, such as a domesticated or wild fowl).

A "therapeutically effective amount" refers to a quantity of a specified agent sufficient to achieve a desired effect in a subject being treated with that agent. Ideally, a therapeutically effective amount of an agent is an amount sufficient to inhibit or treat the disease or condition without causing a substantial cytotoxic effect in the subject. The therapeutically effective amount of an agent will be dependent on the subject being treated, the severity of the affliction, and the manner of administration of the therapeutic composition.

"Treatment" refers to a therapeutic intervention that ameliorates a sign or symptom of a disease or pathological condition after it has begun to develop. As used herein, the term "ameliorating," with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease. The phrase "treating a disease" refers to inhibiting the full development of a disease, for example, in a subject who is at risk for a disease. A "prophylactic" treatment is a treatment administered to a subject who does not exhibit signs of a disease or exhibits only early signs for the purpose of decreasing the risk of developing a pathology or condition, or diminishing the severity of a pathology or condition.

### Methods of Treatment

Disclosed herein is the use of therapeutic agents for improving exercise performance, treating obesity (e.g., inducing weight loss), fatty liver, rhabdomyolysis, Leigh syndrome, other genetic and acquired forms of mitochondrial Complex I deficiency, fatty acid oxidation disorders, lactic acidosis, metformin toxicity, ischemic acute kidney injury, and nephrotoxin-induced kidney injury. The agents may be administered to a subject having, suspected of having, or at risk of developing, obesity (e.g., inducing weight loss), fatty liver, rhabdomyolysis, Leigh syndrome, mitochondrial Complex I deficiency, fatty acid oxidation disorders, lactic acidosis, metformin toxicity, ischemic acute kidney injury, or nephrotoxin-induced kidney injury.

In certain embodiments, the agents are alternative energy sources to their monocarboxylic medium-chain counterparts. In certain embodiments, the subject is in need of, or has been recognized as being in need of, treatment with an alternative energy source as disclosed herein. In certain embodiments, the subject may be in need of a therapeutic agent that bypasses mitochondrial fatty acid oxidation and stimulates peroxisomal fatty acid oxidation.

The therapeutic agents disclosed herein include straight-chain saturated dicarboxylic acids of 8 to 12 carbons in length. In certain embodiments, the saturated dicarboxylic acid is dodecanedioic acid, decanedioic acid (i.e., sebacic acid), octanedioic acid (i.e., suberic acid), undecanedioic acid, or nonanedioic acid (i.e., azelaic acid)**.** In another embodiment, the agents include triglycerides made from the straight-chain saturated dicarboxylic acids. Examples of such triglycerides are shown in FIG 12, for dodecanedioic acid (DC12) and octanedioc acid (DC8). The latter triglyceride of octanedioc acid (Tri-DC8) is a novel compound that has been synthesized at the University of Pittsburgh. DC8 free acid was dissolved in dichloromethane and add to 2-chlorotrityl chloride resin at a volume of approximately 10ml/gm of starting resin. After adding two equivalents of diisopropylethylamine, the compounds were gently mixed @ 300 RPM for 10 minutes at room temperature. Then, an additional three equivalents of diisopropylethylamine were added and the solution mixed for an additional 1 hour @ 300 RPM and room temperature. Then, unreacted resin-bound trityl groups were capped with an excess of neat methanol for 15 minutes at room temperature followed by successive washes with dichloromethane and dimethylformamide. Next, 10 equivalents of 1,2,3-trichloropropane were dissolved in dimethylformamide and added to the DC8-bound 2-chlorotrityl resin while mixing for 2 hours @ 300 RPM at room temperature. Successive washes of the dichloropropane-DC8-2-chlorotrityl intermediate resin were performed with dichloromethane and dimethylformamide. Then, four equivalents of DC8 were added and allowed to mix overnight @ 300 RPM at room temperature. Then, the resin was washed with dichloromethane and dimethylformamide followed by cleavage of the tri-DC8 compound from the resin with 20% 1,1,1,3,3,3-hexafluoro-2-propanol in dichloromethane for 1 hour at room temperature. The solvents were removed on a Buchi Rotavapor unit to form an oily film, and tri-DC8 directly purified by preparative C-18 RP-HPLC on a Waters Delta Prep 4000 chromatography system using standard acetonitrile/0.1% trifluoroacetic acid gradient conditions followed by lyophilization to an oily residue. The purified-lyophilized tri-DC8 compound was dissolved in water and the pH was adjusted to pH 7 using 1N NaOH followed by shell freezing and lyophilization to a powder which was the sodium salt form of the tri-DC8 product. MALDI-TOF analysis of the product on a Bruker Ultraflextreme MS workstation, using α-cyano-4-hydroxycinnamic acid matrix and run in negative ion mode, confirmed the expected mass and identity of the final tri-DC8 product (m/z @ 562amu found_[M+H] = 561amu-calculated). Based on this successful synthesis, the therapeutic agents herein will thus include triglycerides and the sodium salts of triglycerides containing straight-chain dicarboxylic fatty acids 8 to 12 carbons in length. These triglycerides may contain the same dicarboxylic fatty acid in all three positions on the glycerol backbone or a mixture of 8 to 12 carbon dicarboxylic fatty acids in any combination.

In another embodiment, the agents include metabolic intermediates produced during the catabolic chain-shortening of straight-chain dicarboxylic acids 8 to 12 carbons in length, including pathway intermediates containing double-bonds, OH groups, and carbonyl groups. Specifically, these intermediates would include the 2-enoyl, 3-hydroxy, and 3-keto intermediates of straight-chain dicarboxylic acids 8 to 12 carbons in length, such as the intermediates depicted in FIG 13 for octanedioic acid. In another embodiment, the agents include triglycerides and triglyceride salts made from 2-enoyl, 3-hydroxy, and 3-keto intermediates of straight-chain dicarboxylic acids 8 to 12 carbons in length.

In certain embodiments, the agents are used for treating ischemic acute kidney injury (AKI). Specifically, the agents can limit tissue damage to the kidney during AKI, saving lives and preventing the development of chronic kidney diseases later in life.

Metabolic signaling during acute kidney injury has emerged as an exciting and
potentially druggable target to not only reverse but inhibit proximal tubule damage associated with AKI and block the subsequent progression to CKD. The majority of metabolic signaling focuses on the mitochondria and stimulation of this pathway as a potential protective mechanism. However, in certain embodiments, the methods disclosed herein bypass mitochondrial fatty acid oxidation and stimulate peroxisomal fatty acid oxidation in the proximal tubule, thus protecting against various diseases and conditions.

We have discovered that straight-chain saturated dicarboxylic acids are preferentially metabolized by peroxisomes rather than mitochondria. Peroxisomes break down straight-chain saturated dicarboxylic acids into smaller metabolites that can transfer to the mitochondria. In particular, acetyl-CoA and succinyl-CoA are formed, which are key intermediates in the mitochondrial tricarboxylic acid (TCA) cycle. These may be released from the peroxisome as their free acids, acetate and succinate. Although not bound by any theory, it is believed that administering straight-chain saturated dicarboxylic acids is anaplerotic the mitochondrial TCA cycle, but does so in a manner that is independent of the mitochondrial fatty acid oxidation pathway, which requires several-fold more oxygen than the peroxisomal fatty acid oxidation pathway and also produces large amounts of reactive oxygen species. This concept is supported by experiments (described in more detail below) in which we performed ischemia/reperfusion injury on mice (simulating the ischemia time seen in transplant, shock or trauma leading to AKI) that had been fed this diet for one week prior to the injury. We found that the kidneys showed less damage and had improved kidney function leading to the conclusion that administration of straight-chain saturated dicarboxylic acids protects against kidney injury and the subsequent progression to CKD.

In certain embodiments, the agents are used for treating mitochondrial long-chain fatty acid oxidation disorders (LC-FAODs). The agents bypass the mitochondrial fatty acid oxidation pathway, replenish the TCA cycle via acetate and succinate, and produce energy to improve glucose homeostasis, muscle function, and cardiac function.

In certain embodiments, the agents may be used for treating metformin toxicity or overdose. Metformin toxicity occurs acutely through accidental ingestion of large amounts metformin, or when comorbidities disrupt the metabolism of metformin, such as kidney disease, liver disease, or a severe, acute illness. Metformin toxicity drives glucose utilization while impairing hepatic glucose synthesis, resulting in life-threatening hypoglycemia and lactic acidosis. Mortality from metformin toxicity is high. The agents can reduce or prevent metformin toxicity in a subject undergoing a therapeutic treatment that includes administering metformin. The agent may be co-administered with metformin. In certain embodiments, the agent is administered prior to administration of metformin. In certain embodiments, the agent is administered simultaneously with administration of metformin. In certain embodiments, the agent is administered after administration of metformin. In certain embodiments, the agent is administered after metformin toxicity in the subject is detected.

In certain embodiments, the agents disclosed herein may be used for nutritional supplementation to enhance performance during high-intensity exercise due to suppression of lactate.

The therapeutic agent may be administered orally, parenterally (including subcutaneous injections (SC or depo-SC), intravenous (IV), intramuscular (IM or depo-IM), intrasternal injection or infusion techniques), sublingually, intranasally (inhalation), intrathecally, topically, ophthalmically, or rectally.

In certain embodiments, the agent can be administered to subjects via inclusion in enteral feeding formulas. Such formulas, currently widely used for both for adults and pediatric populations, can be used as either supplemental nutrition or sole-source nutrition. Such formulas would contain a balanced mixture of carbohydrate, fiber, protein, and lipid supplemented with electrolytes and vitamins. Such formulas may include dicarboxylic acids as part of the lipid component.

In some embodiments, the methods disclosed herein involve administering to a subject in need of treatment a pharmaceutical composition, for example a composition that includes a pharmaceutically acceptable carrier and a therapeutically effective amount of one or more of the therapeutic agents disclosed herein. The pharmaceutical composition may be administered in dosage unit formulations containing conventional non-toxic pharmaceutically acceptable carriers, adjuvants, and/or vehicles. The therapeutic agents are preferably formulated into suitable pharmaceutical preparations such as tablets, capsules, or elixirs for oral administration or in sterile solutions or suspensions for parenteral administration. Typically the therapeutic agents described above are formulated into pharmaceutical compositions using techniques and procedures well known in the art.

In some embodiments, one or more of the disclosed therapeutic agents (including therapeutic agents linked to a detectable label or cargo moiety) are mixed or combined with a suitable pharmaceutically acceptable carrier to prepare a pharmaceutical composition. Pharmaceutical carriers or vehicles suitable for administration of the compounds provided herein include any such carriers known to be suitable for the particular mode of administration. Remington: The Science and Practice of Pharmacy, The University of the Sciences in Philadelphia, Editor, Lippincott, Williams, & Wilkins, Philadelphia, PA, 21st Edition (2005), describes exemplary compositions and formulations suitable for pharmaceutical delivery of the compounds disclosed herein. In addition, the therapeutic agents may be formulated as the sole pharmaceutically active ingredient in the composition or may be combined with other active ingredients.

Upon mixing or addition of the therapeutic agents(s) to a pharmaceutically acceptable carrier, the resulting mixture may be a solution, suspension, emulsion, or the like. Liposomal suspensions may also be suitable as pharmaceutically acceptable carriers. These may be prepared according to methods known to those skilled in the art. The form of the resulting mixture depends upon a number of factors, including the intended mode of administration and the solubility of the compound in the selected carrier or vehicle. Where the therapeutic agents exhibit insufficient solubility, methods for solubilizing may be used. Such methods are known and include, but are not limited to, using cosolvents such as dimethylsulfoxide (DMSO), using surfactants such as Tween^{®}, and dissolution in aqueous sodium bicarbonate. Derivatives of the therapeutic agents, such as salts or prodrugs may also be used in formulating effective pharmaceutical compositions. The disclosed therapeutic agents may also be prepared with carriers that protect them against rapid elimination from the body, such as time-release formulations or coatings. Such carriers include controlled release formulations, such as, but not limited to, microencapsulated delivery systems.

The disclosed therapeutic agents and/or compositions can be enclosed in multiple or single dose containers. The therapeutic agents and/or compositions can also be provided in kits, for example, including component parts that can be assembled for use. For example, one or more of the disclosed therapeutic agents may be provided in a lyophilized form and a suitable diluent may be provided as separated components for combination prior to use. In some examples, a kit may include a disclosed therapeutic agent and a second therapeutic agent (such as metformin) for co-administration. The disclosed therapeutic agent and second therapeutic agent may be provided as separate component parts. A kit may include a plurality of containers, each container holding one or more unit dose of the compound. The containers are preferably adapted for the desired mode of administration, including, but not limited to tablets, gel capsules, sustained-release capsules, and the like for oral administration; depot products, pre-filled syringes, ampoules, vials, and the like for parenteral administration; and patches, medipads, creams, and the like for topical administration.

The active therapeutic agent is included in the pharmaceutically acceptable carrier in an amount sufficient to exert a therapeutically useful effect in the absence of undesirable side effects on the subject treated. A therapeutically effective concentration may be determined empirically by testing the agents in known *in vitro* and *in vivo* model systems for the treated disorder. In some examples, a therapeutically effective amount of the agent is an amount that lessens or ameliorates at least one symptom of the disorder for which the compound is administered. Typically, the compositions are formulated for single dosage administration. The concentration of active agent in the drug composition will depend on absorption, inactivation, and excretion rates of the active agent, the dosage schedule, and amount administered as well as other factors known to those of skill in the art.

In some examples, about 0.1 mg to 1000 mg of a disclosed agent, a mixture of such agents, or a physiologically acceptable salt or ester thereof, is compounded with a physiologically acceptable vehicle, carrier, excipient, binder, preservative, stabilizer, flavor, etc., in a unit dosage form. The amount of active substance in those compositions or preparations is such that a suitable dosage in the range indicated is obtained. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient. In some examples, the compositions are formulated in a unit dosage form, each dosage containing from about 1 mg to about 1000 mg (for example, about 2 mg to about 500 mg, about 5 mg to 50 mg, about 10 mg to 100 mg, or about 25 mg to 75 mg) of the one or more agents. In other examples, the unit dosage form includes about 0.1 mg, about 1 mg, about 5 mg, about 10 mg, about 20 mg, about 30 mg, about 40 mg, about 50 mg, about 60 mg, about 70 mg, about 80 mg, about 90 mg, about 100 mg, about 150 mg, about 200 mg, about 250 mg, about 300 mg, about 400 mg, about 500 mg, about 600 mg, about 700 mg, about 800 mg, about 900 mg, about 1000 mg, or more of the disclosed agents(s).

The disclosed agents or compositions may be administered as a single dose, or may be divided into a number of smaller doses to be administered at intervals of time. The therapeutic compositions can be administered in a single dose delivery, by continuous delivery over an extended time period, in a repeated administration protocol (for example, by a multi-daily, daily, weekly, or monthly repeated administration protocol). It is understood that the precise dosage, timing, and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. In addition, it is understood that for a specific subject, dosage regimens may be adjusted over time according to the individual need and the professional judgment of the person administering or supervising the administration of the compositions, and that the concentration ranges set forth herein are exemplary only.

When administered orally as a suspension, these compositions are prepared according to techniques well known in the art of pharmaceutical formulation and may contain microcrystalline cellulose for imparting bulk, alginic acid or sodium alginate as a suspending agent, methylcellulose as a viscosity enhancer, and sweeteners/flavoring agents. As immediate release tablets, these compositions may contain microcrystalline cellulose, dicalcium phosphate, starch, magnesium stearate and lactose and/or other excipients, binders, extenders, disintegrants, diluents and lubricants. If oral administration is desired, the agent is typically provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

Oral compositions will generally include an inert diluent or an edible carrier and may be compressed into tablets or enclosed in gelatin capsules. For the purpose of oral therapeutic administration, the active agent or agents can be incorporated with excipients and used in the form of tablets, capsules, or troches. Pharmaceutically compatible binding agents and adjuvant materials can be included as part of the composition. The tablets, pills, capsules, troches, and the like can contain any of the following ingredients or compounds of a similar nature: a binder such as, but not limited to, gum tragacanth, acacia, corn starch, or gelatin; an excipient such as microcrystalline cellulose, starch, or lactose; a disintegrating agent such as, but not limited to, alginic acid and corn starch; a lubricant such as, but not limited to, magnesium stearate; a gildant, such as, but not limited to, colloidal silicon dioxide; a sweetening agent such as sucrose or saccharin; and a flavoring agent such as peppermint, methyl salicylate, or fruit flavoring.
When the dosage unit form is a capsule, it can contain, in addition to material of the above type, a liquid carrier such as a fatty oil. In addition, dosage unit forms can contain various other materials, which modify the physical form of the dosage unit, for example, coatings of sugar and other enteric agents. The agents can also be administered as a component of an elixir, suspension, syrup, wafer, chewing gum or the like. A syrup may contain, in addition to the active agents, sucrose as a sweetening agent and certain preservatives, dyes and colorings, and flavors.

When administered orally, the agents can be administered in usual dosage forms for oral administration. These dosage forms include the usual solid unit dosage forms of tablets and capsules as well as liquid dosage forms such as solutions, suspensions, and elixirs. When the solid dosage forms are used, it is preferred that they be of the sustained release type so that the compounds need to be administered only once or twice daily. In some examples, an oral dosage form is administered to the subject 1, 2, 3, 4, or more times daily. In additional examples, the agents can be administered orally to humans in a dosage range of 1 to 1000 mg/kg body weight in single or divided doses. One illustrative dosage range is 0.1 to 200 mg/kg body weight orally (such as 0.5 to 100 mg/kg body weight orally) in single or divided doses. For oral administration, the compositions may be provided in the form of tablets containing about 1 to 1000 milligrams of the active ingredient, particularly 1, 5, 10, 15, 20, 25, 50, 75, 100, 150, 200, 250, 300, 400, 500, 600, 750, 800, 900, or 1000 milligrams of the active ingredient. It will be understood, however, that the specific dose level and frequency of dosage for any particular patient may be varied and will depend upon a variety of factors including the activity of the specific agent employed, the metabolic stability and length of action of that agent, the age, body weight, general health, sex, diet, mode and time of administration, rate of excretion, drug combination, the severity of the particular condition, and the host undergoing therapy.

Injectable solutions or suspensions may also be formulated, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid. Solutions or suspensions used for parenteral, intradermal, subcutaneous, or topical application can include any of the following components: a sterile diluent such as water for injection, saline solution, fixed oil, a naturally occurring vegetable oil such as sesame oil, coconut oil, peanut oil, cottonseed oil, and the like, or a synthetic fatty vehicle such as ethyl oleate, and the like, polyethylene glycol, glycerine, propylene glycol, or other synthetic solvent; antimicrobial agents such as benzyl alcohol and methyl parabens; antioxidants such as ascorbic acid and sodium bisulfite; chelating agents such as ethylenediaminetetraacetic acid (EDTA); buffers such as acetates, citrates, and phosphates; and agents for the adjustment of tonicity such as sodium chloride and dextrose. Parenteral preparations can be enclosed in ampoules, disposable syringes, or multiple dose vials made of glass, plastic, or other suitable material. Buffers, preservatives, antioxidants, and the like can be incorporated as required.

Where administered intravenously, suitable carriers include physiological saline, phosphate buffered saline (PBS), and solutions containing thickening and solubilizing agents such as glucose, polyethylene glycol, polypropyleneglycol, and mixtures thereof. Liposomal suspensions including tissue-targeted liposomes may also be suitable as pharmaceutically acceptable carriers.

Certain embodiments are described below in the following numbered paragraphs:
1. A method comprising:
   administering a therapeutically effective amount of a therapeutic agent to a subject for treating obesity, fatty liver, rhabdomyolysis, Leigh syndrome, mitochondrial Complex I deficiency, fatty acid oxidation disorders, lactic acidosis, metformin toxicity, ischemic acute kidney injury, or nephrotoxin-induced kidney injury, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.
2. The method of paragraph 1, wherein the straight-chain saturated dicarboxylic acid is dodecanedioic acid, decanedioic acid, octanedioic acid, undecanedioic acid, or nonanedioic acid.
3. The method of paragraph 1, wherein the straight-chain saturated dicarboxylic acid is octanedioic acid.
4. The method of paragraph 1, wherein the straight-chain saturated dicarboxylic acid is dodecanedioic acid.
5. The method of any one of paragraphs 1 to 4, wherein the method comprises treating ischemic acute kidney injury.
6. The method of any one of paragraphs 1 to 4, wherein the method comprises treating obesity.
7. The method of any one of paragraphs 1 to 4, wherein the method comprises treating metformin toxicity or overdose.
8. The method of paragraph 7, wherein the therapeutic agent is co-administered with metformin.
9. The method of any one of paragraphs 1 to 4, wherein the method comprises treating lactic acidosis.
10. The method of paragraph 9, wherein the method comprises administering the therapeutic agent to the subject as a nutritional supplement for enhancing performance during exercise.
11. The method of any one of paragraphs 1 to 4, wherein the fatty acid oxidation disorder is a mitochondrial long-chain fatty acid oxidation disorder.
12. The method of any one of paragraphs 1 to 4, wherein the method induces weight loss.
13. The method of any one of paragraphs 1 to 12, wherein the therapeutic agent is administered orally.
14. The method of paragraph 13, wherein the therapeutic agent is included in an enteral feeding formula.
15. A method comprising:
   administering a therapeutically effective amount of a therapeutic agent to a subject in need of a therapeutic agent that bypasses mitochondrial fatty acid oxidation and stimulates peroxisomal fatty acid oxidation, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.
16. The method of paragraph 15, wherein the straight-chain saturated dicarboxylic acid is dodecanedioic acid, decanedioic acid, octanedioic acid, undecanedioic acid, or nonanedioic acid.

### Examples

In the examples and figures, "DC8" refers to octanedioic acid, "DC10" refers to decanedioic acid, and "DC12" refers to dodecanedioic acid.

We hypothesized that peroxisomes can chain-shorten DC12 as far as DC4-CoA, also known as succinyl-CoA. Such a mechanism would be predicted to 1) result in protein succinylation in the peroxisome, due to the known high chemical reactivity between lysine residues and succinyl-CoA; and 2) "feed" the mitochondrial TCA cycle with the products of DC12 chain-shortening, namely 2-carbon units (acetate) and succinate. We fed wildtype
C57Bl/6 mice DC12 mixed into the diet and measured protein succinylation as a proxy for succinyl-CoA formation. Western blotting with a pan antisuccinyllysine antibody revealed a
massive upregulation of this PTM in the liver and kidney (FIG. 1A), the two organs most rich in peroxisomes, while muscle, heart, and brain showed no change in succinylation. The succinylation is seen to arise within 1 day and becomes maximal at 7 days (FIG. 1B). The effect is reversible when the diet is removed. DC12 degradation also produces acetyl-CoA. However, this metabolite is much less reactive than succinyl-CoA, and no changes were observed in protein acetylation using a pan anti-acetyllysine antibody. Like succinyl-CoA, glutaryl-CoA (DC5-CoA) is also highly reactive. As further demonstration that DCA diets can change the PTM landscape, we fed mice 10% DC11 for 7 days, predicting it would be chain-shortened two carbons at a time to DC5, causing protein glutarylation. Indeed, DC11 induce high-level protein glutarylation in the liver and kidney (FIG. 1C).

An important question was whether Sirt5, a deacylase that can reverse succinylation and glutarylation, would offset and reverse the effects of feeding DCAs. Purified peroxisomes from the livers of wildtype and Sirt5KO mice fed 10% DC12 for 7 days showed similar patterns of lysine succinylation (FIGS. 2A, 2B). Quantitative mass spectrometry of these livers confirmed that 1) feeding DC12 increases lysine succinylation dramatically on peroxisomal FAO proteins, and 2) that deletion of Sirt5 does not enhance this effect (FIG. 2C). Interestingly, this data indicates that while Sirt5 deletion by itself has been shown to be renoprotective and increase peroxisomal FAO, it is not due to direct action on peroxisomal proteins.

DC12 is renoprotective. Based on our findings detailed above, we hypothesized that feeding DC12 would prove bioenergetically favorable in the kidney during ischemic injury. To test this, mice that had been fed ± 10% DC12 for 7 days were subjected to unilateral ischemic
kidney injury. In this model, the uninjured contralateral kidney is removed on Day 6 post-injury and used as control tissue, and the injured kidneys are collected one day later for analysis. The diet was continued during the 6-day recovery period post-injury, as well as after removing the contralateral kidney. As indicated by H&E staining, feeding DC12 reduced the number of dilated tubules and proteinaceous casts post-injury (FIGS. 3A,3B). Further, immunostaining revealed less induction of the injury marker NGAL (FIGS. 3C,3D). This was further coupled with significantly reduced serum creatinine and blood urea nitrogen (BUN), which are global
markers of kidney dysfunction (FIGS. 3E,3F).

Portions of contralateral control kidney and injured kidney were used for quantitative mass spectrometry, to measure both total protein abundance and site-level changes in lysine succinylation. The DC12 diet had very minimal if any effect on protein expression (FIG. 4A), but massive effects on lysine succinylation (FIG. 4B). Ischemic AKI reduced the succinylation
level of kidney peroxisomal proteins compared to baseline (contralateral), while DC12 feeding greatly increased it (FIG. 4C).

We also conducted a nephrotoxic model of AKI (cisplatin). DC12 similarly protected against injury in this model. We focused on the tubular marker biocarbonate which showed improved functionality in the DC12 fed animals compared with the controls and less structural damage histologically (FIG. 5).

FIG. 6 shows data directly comparing DC8, DC10, and DC12. DC12 induces the strongest protein succinylation in liver. In kidney, DC8 and DC10 induced stronger protein succinylation. DC8 induces high-level, kidney-specific protein succinylation. This suggests that
DC8 is not metabolized by liver and can therefore be utilized as a kidney-targeted therapy. Potential side effects caused by liver metabolization can be avoided, and the liver is not reducing the available agent.

The effect of DC8 in ischemic AKI was also tested. Here we observe a marked reduction in kidney damage in the DC8 treated animals compared to the controls (FIG. 7) and even in comparison to DC12 treated animals (see FIG. 3). Kidney function was also
dramatically improved in the DC8 treated compared to either untreated controls or DC12-fed animals. Further to this, we also tested DC8 in a nephrotoxic (cisplatin) model of AKI and showed similar histological and functional protection.

Taken together this shows that DC8 improves the kidney protection and reduces potential off target effects.

In view of the many possible embodiments to which the principles of the disclosed invention may be applied, it should be recognized that the illustrated embodiments are only preferred examples of the invention and should not be taken as limiting the scope of the invention.
**The present application and invention further includes the subject matter of the following numbered clauses:**
1. A method comprising:
   administering a therapeutically effective amount of a therapeutic agent to a subject for treating obesity, fatty liver, rhabdomyolysis, Leigh syndrome, mitochondrial Complex I deficiency, fatty acid oxidation disorders, lactic acidosis, metformin toxicity, ischemic acute kidney injury, or nephrotoxin-induced kidney injury, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.
2. The method of clause 1, comprising administering the straight-chain saturated dicarboxylic acid of 8 to 12 carbons in length, wherein the straight-chain saturated dicarboxylic acid is dodecanedioic acid, decanedioic acid, octanedioic acid, undecanedioic acid, or nonanedioic acid.
3. The method of clause 1, comprising administering the straight-chain saturated dicarboxylic acid of 8 to 12 carbons in length, wherein the straight-chain saturated dicarboxylic acid is octanedioic acid.
4. The method of clause 1, comprising administering the straight-chain saturated dicarboxylic acid of 8 to 12 carbons in length, wherein the straight-chain saturated dicarboxylic acid is dodecanedioic acid.
5. The method of clause 1, comprising administering the triglyceride made from a straight-chain saturated dicarboxylic acid of 8 to 12 carbons in length, wherein the triglyceride is a triglyceride of dodecanedioic acid, a triglyceride of decanedioic acid, a triglyceride of octanedioic acid, a triglyceride of undecanedioic acid, a triglyceride of nonanedioic acid, or a salt thereof.
6. The method of clause 1, comprising administering the triglyceride made from a straight-chain saturated dicarboxylic acid of 8 to 12 carbons in length, wherein the triglyceride is a triglyceride of octanedioic acid, or a salt thereof.
7. The method of clause 1, comprising administering the metabolic intermediate produced during the catabolic chain-shortening of a straight-chain dicarboxylic acid of 8 to 12 carbons in length, wherein the intermediate is a 2-enoyl intermediate of a straight-chain dicarboxylic acid 8 to 12 carbons in length , a 3-hydroxy intermediate of a straight-chain dicarboxylic acid 8 to 12 carbons in length, or a 3-keto intermediate of a straight-chain dicarboxylic acid 8 to 12 carbons in length.
8. The method of any one of clauses 1 to 7, wherein the method comprises treating ischemic acute kidney injury.
9. The method of any one of clauses 1 to 7, wherein the method comprises treating obesity.
10. The method of any one of clauses 1 to 7, wherein the method comprises treating metformin toxicity or overdose.
11. The method of clause 10, wherein the therapeutic agent is co-administered with metformin.
12. The method of any one of clauses 1 to 7, wherein the method comprises treating lactic acidosis.
13. The method of clause 12, wherein the method comprises administering the therapeutic agent to the subject as a nutritional supplement for enhancing performance during exercise.
14. The method of any one of clauses 1 to 7, wherein the fatty acid oxidation disorder is a mitochondrial long-chain fatty acid oxidation disorder.
15. The method of any one of clauses 1 to 7, wherein the method induces weight loss.
16. The method of any one of clauses 1 to 15, wherein the therapeutic agent is administered orally.
17. The method of clause 16, wherein the therapeutic agent is included in an enteral feeding formula.
18. A method comprising:
   administering a therapeutically effective amount of a therapeutic agent to a subject in need of a therapeutic agent that bypasses mitochondrial fatty acid oxidation and stimulates peroxisomal fatty acid oxidation, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.
19. The method of clause 18, wherein the straight-chain saturated dicarboxylic acid is dodecanedioic acid, decanedioic acid, octanedioic acid, undecanedioic acid, or nonanedioic acid.
20. A method comprising:
   administering a therapeutically effective amount of a therapeutic agent to a subject for improving exercise performance, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.
21. A method comprising:
   administering a therapeutically effective amount of a therapeutic agent to a subject for treating fibrosis in chronic kidney disease, wherein the therapeutic agent is a straight-chain saturated dicarboxylic acid of 8 to 12 carbons in length, a triglyceride made from a straight-chain saturated dicarboxylic acid of 8 to 12 carbons in length, a metabolic intermediate produced during the catabolic chain-shortening of a straight-chain dicarboxylic acid of 8 to 12 carbons in length, a salt thereof, or a mixture thereof.

## Claims

1. A therapeutic agent for use in a method of treating a disease or disorder;
wherein said disease or disorder is selected from the group consisting of lactic acidosis, fatty liver, rhabdomyolysis, Leigh syndrome, mitochondrial complex 1 deficiency, fatty acid oxidation disorder, metformin toxicity, ischemic acute kidney injury, nephrotoxin-induced kidney injury and fibrosis in chronic kidney disease;
wherein the therapeutic agent is selected from: dodecanedioic acid, decanedioic acid, octanedioic acid, a triglyceride of dodecanedioic acid, a triglyceride of decanedioic acid, a triglyceride of octanedioic acid, or a salt thereof;
wherein the use comprises administering a therapeutically effective amount of the therapeutic agent to a subject.

2. The agent for use of claim 1, wherein the therapeutic agent is selected from:
dodecanedioic acid, decanedioic acid, or octanedioic acid, or a salt thereof.

3. The agent for use of claim 1, wherein the therapeutic agent is octanedioic acid.

4. The agent for use of claim 1, wherein the therapeutic agent is dodecanedioic acid.

5. The agent for use of claim 1, wherein the therapeutic agent is selected from a triglyceride of dodecanedioic acid, a triglyceride of decanedioic acid, a triglyceride of octanedioic acid, or a salt thereof.

6. The agent for use of claim 1, wherein the therapeutic agent is a triglyceride of octanedioic acid, or a salt thereof.

7. The agent for use of any one of claims 1 to 6, wherein the disease or disorder is lactic acidosis.

8. The agent for use of claim 7, wherein the use comprises administering the therapeutic agent to the subject as a nutritional supplement for enhancing performance during exercise.

9. The agent for use of any one of claims 1 to 6, wherein the disease or disorder is ischemic acute kidney injury.

10. The agent for use of any one of claims 1 to 6, wherein the disease or disorder is metformin toxicity or overdose.

11. The agent for use of claim 10, wherein the therapeutic agent is co-administered with metformin.

12. The agent for use of any one of claims 1 to 11, wherein the therapeutic agent is administered orally.

13. The agent for use of claim 12, wherein the therapeutic agent is included in an enteral feeding formula.
